# EUROPEAN PATENT SPECIFICATION

(11) **EP 3 741 289 B1**
(45) Date of publication and mention of the grant of the patent: **30.08.2023**
(21) Application number: 19175395.3
(22) Date of filing: 20.05.2019
(51) Int. Cl.: A61B 5/00, A61B 5/087, A61M 16/00, A61M 15/00

(54) **NEBULIZATION DEVICE COMPRISING AN APPARATUS FOR CHECKING AND CONTROLLING BREATHING OF A PATIENT**
VERNEBELUNGSVORRICHTUNG MIT EINEM GERÄT ZUR KONTROLLE UND STEUERUNG DER ATMUNG EINES PATIENTEN
DISPOSITIF DE NÉBULISATION COMPRENANT UN APPAREIL DE SURVEILLANCE ET DE CONTRÔLE DE LA RESPIRATION D'UN PATIENT

(43) Date of publication of application: 25.11.2020
(73) Proprietor: Flaem Nuova S.p.A., 25015 Desenzano del Garda (BS) (IT)
(72) Inventor: Abate, Riccardo, 25015 Desenzo del Garda (BS) (IT); Ongaretti, Luca, 25060 Cellatica (BS (IT); Bertelli, Mauro, 25128 Brescia (IT)
(74) Representative: Botti & Ferrari S.p.A.

(56) References cited:
- WO-A1-2017/178865
- US-A- 5 794 612
- US-A1- 2013 008 436
- US-A1- 2019 060 590

## Description

### Field of application

The present invention relates to a nebulization device comprising an apparatus for checking and controlling the correct breathing, said apparatus being associated with a device for inhalation therapies by a patient suffering from diseases of the upper respiratory tract.

The above apparatus finds useful application in the field of nebulization devices.

### Prior art

The use of inhaler devices is known as a therapeutic practice in connection with several diseases of the respiratory system is known.

Therefore, several inhalers have been developed, which allow obtaining the undoubted advantage of reaching high concentrations of drugs, directly in the airways, without all the side effects of the systemic administration. Various methods of inhaling drugs are available: nebulization devices or nebulizers, pre-dosed sprays (MDI) and dry powder inhalers (DPI).

However, the inhaled drugs' effectiveness is affected by various factors, one of the most important of which is the correct execution of the inhalation therapy.

In fact, inhalers, and in particular the nebulization devices, ensure an effective intake of the drug if used appropriately, which often does not occur because of an inaccurate use or simply due to the lack of the patient's adequate competence.

In this regard, inhalers provided with an integrated control system capable of interacting with the patient and of checking the correct execution of the therapy thereof have recently been developed.

These devices are clearly very complex, sometimes not totally intuitive and particularly expensive, in addition to requiring the replacement of devices without these features, but fully functional.

For this reason, the need is felt to have a clear indication about the correct application of the established therapeutic indications and meanwhile to allow the use of any inhaler device, or nebulization device, whether or not provided with an integrated control system.

International patent application n. WO 2018/160073 A1, relating to an adapter applicable to a dispenser for a powder drug using a pressure sensor, is directed in this regard. Though being a practice device, it is not particularly accurate in detecting the patient's breathing, thus not ensuring a precise checking of the therapeutic activity performed by the patient himself.

An alternative is provided by International patent application n. WO 2017/180980 A1, which describes a method and an adapter that provide for a passage in which a timer is triggered from the moment when this determined physical parameter exceeds a threshold value until such physical parameter falls below the threshold value. However, this solution is complicated by the need of installing a correct threshold parameter for a specific application.

Yet the device described in European patent application n. EP 3 129 088 A1 relating to a device for educating to the correct inhalation, comprising a microphone which evaluates the passing air flow. This device allows a superficial control detecting macroscopic errors, and therefore it is not suitable for an accurate control of the inhalation therapy execution.

WO 2017/ 178865 A1 discloses an inhaler add-on device with a system of improving compliance with a specified use with at least one pressure sensor and a component of a mask configured to removably fit with an outer surface of the mask.

US 5,794,612 A discloses an intrapulmonary device having an indication system with a sensor that responds to a selected range of ultrasound, a sensor associated controller, a differential pressure sensor and a display for signals. The controller is associated with a microprocessor whose data is stored in memory.

US 2013/008436 A1 discloses a medical device including a feedback device with one or more sensors for detecting a parameter relating to the use of the apparatus and a processing unit for providing feedback information to a patient.

An object of the present invention is to conceive an apparatus capable of overcoming the above prior art drawbacks, and in particular allowing checking and controlling the breathing activity performed by a patient in an inhalation therapy execution.

Another object is to provide an apparatus which can be used in a highly intuitive manner by any patient without the need for a specific and accurate preventive preparation.

A further object is to provide an apparatus capable of also communicating with remote devices in order to enhance their operational functionality. Finally, a further object is to provide a structurally simple apparatus to avoid construction problems not suitable for a product intended for mass production.

### Summary of the invention

The invention is defined by independent claim 1, preferred embodiments are defined by the dependent claims.

The solution idea underlying the present invention is to make an apparatus associable with device for inhalation therapy and to provide an output related to the patient's detected breathing in the inhalation therapy phase, so as to check the correct execution thereof.

The above technical problem is solved by a nebulization device comprising an apparatus for checking and controlling breathing of a patient, said apparatus comprising a connection element for connecting said apparatus with a patient's respiratory tract, a main body provided with an integrated display device, one or more sensors adapted to continuously detect during the inhalation therapy a variation of at least one physical quantity associated with an air flow inhaled and/or exhaled by the patient, passing through said connection element and susceptible of emitting at least one signal related to said variation. The apparatus further comprises a control system integrated in the apparatus and configured to receive said signal to perform a correspondence check of said at least one signal with at least one pre-set parameter in said control system, and emit an error signal in case of non-correspondence between said at least one signal with said at least one pre-set parameter, said pre-set parameter being obtained by said control system by an initial screening of the patient's breath through an analysis of a plurality of breaths, said pre-set parameter being compared during the course of each single delivery of said nebulization device to the signal detected by said one or more sensors.

Advantageously, the one or more sensors comprise at least one pressure sensor adapted to detect a pressure of the air flow inhaled and/or exhaled by a patient, passing through the connection element and susceptible of emitting at least one pressure-related signal.

Advantageously, such an apparatus allows checking in real time and continuously the air flow inhaled and/or exhaled by a patient during the inhalation therapy, said identification and signal-response functions being performed jointly and in a coordinated manner by the pressure sensor and by the control system. Therefore, it is immediately possible to send an output information to the patient or continuously or just under certain alert conditions by the integrated display device.

In a still preferred embodiment, the at least one pressure sensor is a differential pressure switch. Such a solution allows an initial self-calibration phase of the apparatus to adapt to the use conditions, for example to altitudes.

Preferably, the apparatus of the nebulization device further comprises a quick fastening system, adapted to allow a connection with said nebulization device.

Advantageosly, this solution makes the use of the device practice, making the apparatus integral with the device which is associated with and allowing the use with only one hand by the patient, as if it were a single nebulization device, all without the need for a global replacement of the nebulization device itself.

In a preferred embodiment, said quick fastening system provides for instance for a snap connection by a clip with a contrast spring.

This fastening system is particularly robust, making the integral connection between the devices particularly safe even in the event the assembly is turned upside down.

In an alternative embodiment the fastening system provides for an attachment strap made of elastic material or Velcro.

Advantageously, the present embodiment is particularly flexible and simple to produce, in addition to being very simple to replicate and replace in the event of damage of the connection or wear.

Preferably, the control system of the apparatus of the nebulization device further comprises a data connection module for a communication and remote control of the apparatus.

Advantageously, this allows a combined operation of the apparatus with external systems, as well as checking a series of parameters, even without being in direct contact with the apparatus, for instance in the event these parameters should be displayed by a third party in addition to the patient.

Still preferably, the apparatus of the nebulization device comprises a check and control remote device for processing at least one signal, the remote device being connected to the apparatus by the data connection module. For instance, said remote device may be constituted by a more complex medical system.

Advantageously this allows increasing the potentiality of computing power and visualization of the device, in addition to providing the chance of storing a history of the information relating to the therapy into an external data repository, for checking an improvement of the execution to be related to an increase in the benefits obtained.

In a preferred embodiment the connection element is connected to the apparatus by a connection tube, preferably a removable one. It is possible to adopt a membrane filter on said connection tube for anticontamination, so in order to protect the pressure sensor from possible nebulized residues such as drugs or excipients.

Alternatively, it is possible not to adopt said connection tube, by providing a different conformation of the apparatus which allows a direct passage of the air flow through the apparatus itself.

Furthermore, according to a preferred embodiment the apparatus of the nebulization device comprises a network connection port for the connection of the apparatus to an external system.

Advantageously this allows a direct connection, for instance to an external PC, for a functional connection or even merely for recharging reasons.

Preferably, the apparatus of the nebulization device further comprises a configuration button, for turning on or off or for the configuration of specific apparatus settings.

Advantageously, this allows a greater functional autonomy of the apparatus.

Still preferably, the apparatus of the nebulization device comprises a battery power system.

Preferably, but not exclusively, lithium ion batteries may be used, which ensure a prolonged use, ideal for a portable apparatus.

Further features and advantages will become clearer from the following detailed description of a preferred, but not exclusive, embodiment of the present invention, with reference to the enclosed figures given by way of non-limiting example.

### Brief description of the drawings

In these drawings:

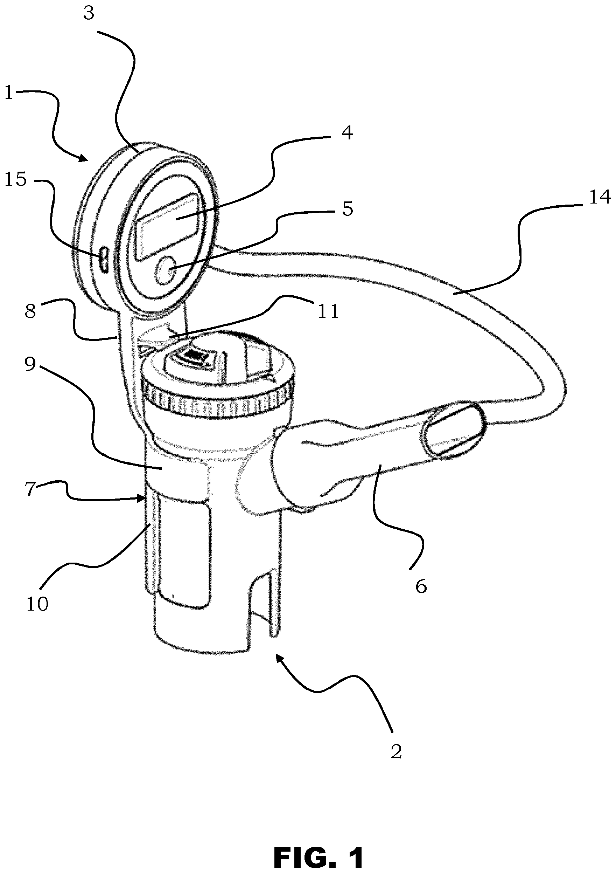

- Figure 1 represents a perspective view of a nebulization device comprising an apparatus for checking and controlling breathing according to the present invention;

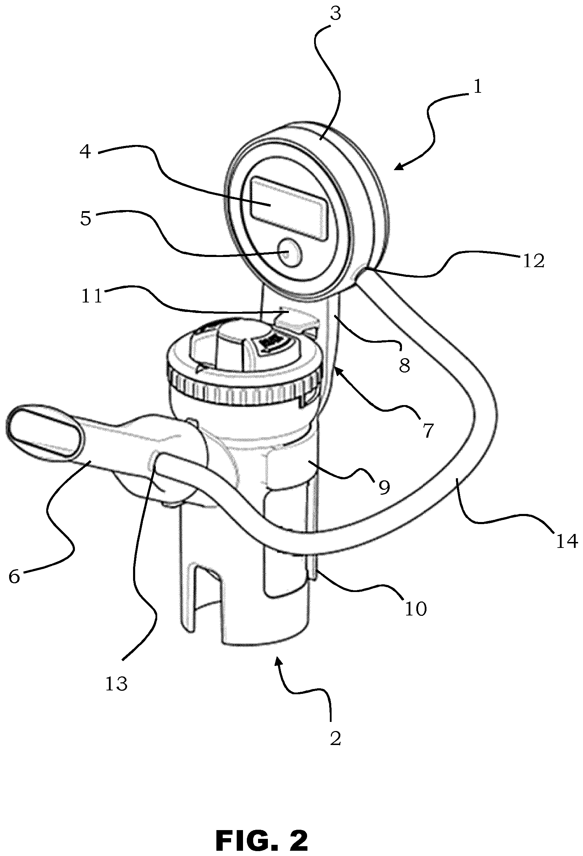

- Figure 2 represents a further perspective view of the nebulization device comprising an apparatus of Figure 1.

### Detailed description

With reference to the Figures, reference number 1 globally and schematically indicates an apparatus for checking and controlling breathing, made according to the present invention, included in a nebulization device or ampoule 2.

The apparatus 1 comprises a main body 3 provided with an integrated display device 4, for displaying information in real time. The integrated display device 4 in the present exemplary and non-limiting embodiment is constituted by an LCD liquid crystal display. Nothing prevents from adopting a dot-matrix or, differently, a LED display, or other types of display devices, all included in the scope defined in the appended claims. Preferably on the main body 3 at least one configuration button 5, for turning on or off or for the configuration of specific apparatus 1 settings is provided.

Furthermore, signal notification devices emitting a tactile type signal, or a sound type signal are possible.

The apparatus 1 further comprises a connection element 6 for connecting with a patient's respiratory tract.

In the present embodiment by way of example the connection element 6 is constituted by a mouthpiece, but nothing prevents from using different connection elements 6, such as nasal forks, masks, or other elements adapted to be passed through a flow inhaled or exhaled by a patient in an inhalation therapy.

The connection element 6 is connected to the nebulization device 2.

The apparatus 1 further comprises one or more sensors (not shown) adapted to detect a variation of at least one physical quantity associated with an air flow inhaled and/or exhaled by the patient, passing through the connection element 6 and susceptible of emitting at least one signal related to said variation. Preferably the one or more sensors comprise at least one pressure sensor (not shown) adapted to detect a pressure of an air flow passing through the apparatus 1, inhaled and/or exhaled by a patient, and adapted to the variation of at least one signal related to said variation. In the continuation of the description particular reference will be made to the embodiment with said pressure sensor, said embodiment being exemplifying and not limiting of the scope of protection defined by the appended claims. Preferably the pressure sensor is constituted by a differential pressure switch, which allows an initial self-calibration phase of the apparatus to adapt to the use conditions, for instance to the altitudes.

Furthermore, the apparatus 1 provides for an integrated control system (not shown), provided with an internal electronic card and adapted to identify the signal of the pressure sensor, to perform the correspondence check of the at least one signal with at least one pre-set parameter stored in the control system, to send an error signal in the event of non-correspondence between the at least one signal with the at least one pre-set parameter. In particular, it is possible to collect and monitor data pertaining to respiratory rate, lung capacity, abnormal breathing interruptions, etc.

The internal electronic card further preferably provides for a data connection module. Such data connection module may be, by way of example, of the Wi-Fi, Bluetooth, Bluetooth Low Energy (BLE) type, or others. The remote connection module allows a combined operation of the apparatus with external systems. In particular, preferably, the data connection module allows the connection of the apparatus to a remote checking and controlling device for a processing of the at least one signal This allows increasing the potentiality of computing power and display of the apparatus 1, in addition to providing the chance of storing data in an external data repository. It is also possible to provide a connection for sending a push notification to an associated mobile device relating to a detected or processed signal.

Furthermore, a network connection port 15 is preferably provided. The connection port 15 may be, for instance, of the USB or micro-USB type and allows the direct connection of the apparatus 1 to an external system, for instance, to an external PC, for a functional connection or merely for recharging reasons.

The apparatus 1 is, in fact, further preferably provided with a battery power system, battery which may be for instance of the lithium ion type, for reasons of longer charge period of the apparatus 1 in conditions of lack of network sockets.

Preferably, the apparatus 1 comprises a quick fastening system 7. The quick fastening system 7 in the present exemplary embodiment is represented by a snap mechanism by a clip in cylindrical coupling.

As visible in the Figures, the apparatus 1 provides for a bearing structure 8, essentially constituted by a parallelepiped portion, which is longitudinal to the axis of the nebulization device 2 and integrally connected to a side surface of the main body 3. Preferably the bearing structure 8 is shaped so to be able to hook the main body 3 in the vertical, upright, or protruding position what to say, with the display device 4 facing towards the connection device 6 and towards the patient. In this way the information reported by the apparatus 1 are made immediately displayable to the patient in the execution phase of the therapy and it is possible to perform the appropriate corrections in real time.

The quick fastening system 7 provides for a clip portion with a "C"-shaped or strap fastening portion 9 adapted to grasp the nebulization device, with a gripping force determined by the elasticity of the material. The quick fastening system 7 further preferably provides for at least one longitudinal portion 10, which is relative to the axis of the nebulization device, integrally connected to the "C"-shaped or strap portion 9, to confer greater structural solidity to the fastening system 7. Still preferably the fastening system 7 provides for a security L-shaped tongue 11, on the bearing structure 8, which hooks at the upper portion of the nebulization device 2, so as to make the apparatus 1 more integral with the nebulization device 2.

Nothing prevents from adopting a different fastening system 7.

For instance, it is possible to provide for an attachment strap made of elastic material or Velcro, a solution which is particularly flexible, simple to produce and easy to replace in the event of damage to the connection or wear.

Alternatively, it is possible to think of a crown connection, which fits above the nebulization device 2. In this case, being an air inflow into the nebulization device 2 necessary, in order for the characteristic internal Venturi system to work properly, a distal passage is obtained inside the apparatus 1 towards the inside of the nebulization device 2 itself. Possibly this distal passage further comprises a valve system.

Other types of connection between the apparatus 1 and the nebulization device 2 are also possible, all falling within the scope of the appended claims.

In the present exemplifying embodiment, the apparatus 1 comprises an air inlet 12. Furthermore, the connection element 6 comprises an air outlet 13. The air inlet 12 and the air outlet 13 are placed in communication by a connection tube 14, preferably a removable one. The connection tube 14 may be made of various materials, such as PVC, TPU, silicone, or other materials suitable for the purpose and comprised in the claimed scope. It is preferable, in this embodiment to arrange a membrane filter on the connection tube 14 to safeguard the pressure sensor from possible nebulized residues such as drugs or excipients.

It is possible to make the air outlet 13 at another point of the flow passage. Other embodiments are further possible, wherein the connection tube 14 is not necessary, since a conformation allowing a direct passage of the air flow through the apparatus 1 is provided.

The operation of the universal apparatus 1 for checking and controlling breathing according to the invention will be described hereinafter, considering, for illustrative purposes, the above described embodiments.

First of all, the apparatus 1 is connected in a functional way to the nebulization device 2 to be monitored.

During the use of the nebulization device 2, through the connection tube 14, the alternation of positive and negative pressure applied and received by the apparatus through the pressure sensor allows checking whether the patient is in the inhalation or exhalation phase, collecting and analyzing a series of data, among which it is possible to cite, only by way of example, respiratory rate, lung capacity, possible abnormal interruption of breath or of therapy. Additionally, it is possible to perform a simple counting of breaths.

The use of a differential pressure switch as pressure sensor allows performing, in the start-up phase of the apparatus 1 functionality, a self-calibration of the apparatus 1 itself, allowing an adaptation to the different conditions in which the operation takes place, in particular, for instance, to the different altitudes of use.

The apparatus 1 does not interact with the nebulization device 2, that is expected to constantly remain in operation during the delivery of the drug or of the nebulized product.

First of all, the apparatus 1, once started, analyses the patient's breath, a procedure for which generally no more than 10 breaths are sufficient, which are performed slowly and in a relaxed manner, without forcing. Such an initial screening allows evaluating the patient's lung capacity and therefore being able to control it during the course of the single delivery. It is also possible to provide for a subsequent or incremental self-calibration to better adhere to the patient's respiratory course.

During breathing the pressure sensor detects and identifies variations which are compared by the control system with the parameters pre-set or imported into the initial screening.

In the event of breath inconsistencies, the apparatus 1 produces an output to the patient, through own display device. Furthermore, a signal of the tactile type or a signal of the sound type are possible. It is also possible to provide for sending a push notification to an associated mobile device. The inconsistencies may be of various types: an interruption of breath or of therapy, cases of hypoventilation or hyperventilation or others.

The data and inconsistencies may be further sent, stored, displayed and processed by the remote device both by the patient himself and by a third party, for instance an attending physician.

Advantageously, a nebulization device comprising an apparatus according to the invention solves the problems occurring in the prior art devices. In particular, the apparatus 1 allows a precise checking and careful control of the respiratory activity performed by a patient in the execution of an inhalation therapy.

There is also to say that the apparatus 1 is of the simple and highly intuitive type for the patient, since it does not require a careful preparation, which also protects from possible use mistakes that could compromise both the correct use of the apparatus 1, and, in more serious cases, the incorrect execution of the inhalation therapy.

Still advantageously, the apparatus 1 may also be placed in communication with a remote device, as well as with a mobile device or a PC, so as to increase the potentiality of the apparatus, to provide a greater number of information, a historical trend of the detected features, and in general to increase the number of implementable operational functionality in the apparatus 1 itself.

A skilled person will also appreciate that the nebulization device 2 comprising the apparatus 1 is structurally simple, avoiding construction problems that are not suitable for a product intended for mass production.

The skilled person will understand that the illustrated embodiment may undergo various changes and variations, according to specific and contingent needs, all included within the scope of protection of the invention, as defined by the following claims.

## Claims

1. Nebulization device (2) comprising an apparatus (1) for checking and controlling breathing of a patient, said apparatus (1) comprising:
- a connection element (6) for connecting said apparatus (1) with a patient's respiratory tract;
- a main body (3) provided with an integrated display device (4);
- one or more sensors adapted to continuously detect during the inhalation therapy a variation of at least one physical quantity associated with an air flow inhaled and/or exhaled by the patient, passing through said connection element (6) and susceptible of emitting at least one signal related to said variation;
- a control system integrated in the apparatus and configured to receive said signal to perform a correspondence checking of said at least one signal with at least one pre-set parameter stored in said control system, and emit an error signal in case of non-correspondence between said at least one signal and said at least one pre-set parameter, said pre-set parameter being obtained by said control system by an initial screening of the patient's breath through an analysis of a plurality of breaths, said pre-set parameter being compared during the course of each single delivery of said nebulization device (2) to the signal detected by said one or more sensors.

2. Nebulization device (2) according to claim 1, wherein said one or more sensors of said apparatus (1) comprise at least one pressure sensor adapted to detect a pressure of the air flow inhaled and/or exhaled by a patient, passing through said connection element (6) and susceptible of emitting at least one signal related to said pressure.

3. Nebulization device (2) according to claim 2, wherein said at least one pressure sensor is of the differential pressure switch type.

4. Nebulization device (2) according to any one of claims 1 to 3, wherein said apparatus (1) further comprising a quick clip fastening system (7), adapted to allow a snap connection (7) with said nebulization device (2).

5. Nebulization device (2) according to claim 4, wherein said quick fastening system (7) provides a snap connection by an elastic fastening "C"-shaped portion.

6. Nebulization device (2) according to claim 4, wherein said quick fastening system (7) provides for an attachment strap made of elastic material or Velcro.

7. Nebulization device (2) according to any one of claims 1 to 6, wherein said control system further comprises a data connection module for a remote communication and control of said apparatus (1).

8. Nebulization device (2) according to claim 7, wherein said apparatus (1) further comprising a checking and control remote device for processing said at least one signal, said remote device being connected to said apparatus (1) by said data connection module.

9. Nebulization device (2) according to any one of claims 1 to 8, wherein said connection element (6) of said apparatus (1) is connected by a connection tube (14).

10. Nebulization device (2) according to claim 2 and 9, wherein said connection tube (14) of said apparatus (1) comprises a membrane filter to protect said pressure sensor from possible nebulized residues such as drugs or excipients.

11. Nebulization device (2) according to any one of claims 1 to 10, wherein said apparatus (1) further comprising a network connection port (15) for connecting said apparatus (1) to an external system.

12. Nebulization device (2) according to any one of claims 1 to 11, wherein said apparatus (1) further comprising at least one configuration button (5).

13. Nebulization device (2) according to any one of claims 1 to 12, wherein said apparatus (1) further comprising a battery power system.

## Patentansprüche

1. Vernebelungsvorrichtung (2) mit einer Vorrichtung (1) zur Überwachung und Kontrolle der Atmung eines Patienten, wobei die Vorrichtung (1) umfasst:
- ein Verbindungselement (6) zum Verbinden der Vorrichtung (1) mit den Atemwegen eines Patienten;
- einen Hauptkörper (3), der mit einer integrierten Anzeigevorrichtung (4) versehen ist;
- einen oder mehrere Sensoren, die geeignet sind, während der Inhalationstherapie kontinuierlich eine Veränderung mindestens einer physikalischen Größe zu erfassen, die mit einem vom Patienten eingeatmeten und/oder ausgeatmeten Luftstrom verbunden ist, der durch das Verbindungselement (6) hindurchgeht, und die geeignet sind, mindestens ein auf diese Veränderung bezogenes Signal abzugeben;
- ein in das Gerät integriertes Steuersystem, das so konfiguriert ist, dass es das Signal empfängt, um eine Überprüfung der Übereinstimmung des mindestens einen Signals mit mindestens einem voreingestellten Parameter, der in dem Steuersystem gespeichert ist, durchzuführen, und ein Fehlersignal ausgibt, falls das mindestens eine Signal und der mindestens eine voreingestellte Parameter nicht übereinstimmen, wobei der voreingestellte Parameter von dem Steuersystem durch ein anfängliches Screening des Atems des Patienten durch eine Analyse einer Vielzahl von Atemzügen erhalten wird, wobei der voreingestellte Parameter während des Verlaufs jeder einzelnen Abgabe der Vernebelungsvorrichtung (2) mit dem von dem einen oder den mehreren Sensoren erfassten Signal verglichen wird.

2. Vernebelungsvorrichtung (2) nach Anspruch 1, wobei der eine oder die mehreren Sensoren der Vorrichtung (1) mindestens einen Drucksensor umfassen, der geeignet ist, einen Druck des von einem Patienten eingeatmeten und/oder ausgeatmeten Luftstroms zu erfassen, der durch das Verbindungselement (6) hindurchgeht, und der geeignet ist, mindestens ein auf den Druck bezogenes Signal zu senden.

3. Vernebelungsvorrichtung (2) nach Anspruch 2, wobei der mindestens eine Drucksensor von der Art ein Differenzdruckschalter ist.

4. Vernebelungsvorrichtung (2) nach einem der Ansprüche 1 bis 3, wobei die Vorrichtung (1) ferner ein Schnellclip-Befestigungssystem (7) umfasst, das geeignet ist, eine Schnappverbindung (7) mit der Vernebelungsvorrichtung (2) zu ermöglichen.

5. Vernebelungsvorrichtung (2) nach Anspruch 4, wobei das Schnellclip-Befestigungssystem (7) eine Schnappverbindung durch einen elastischen "C"-förmigen Befestigungsabschnitt bereitstellt.

6. Vernebelungsgerät (2) nach Anspruch 4, wobei das Schnellclip-Befestigungssystem (7) ein Befestigungsband aus elastischem Material oder Klettverschluss vorsieht.

7. Vernebelungsgerät (2) nach einem der Ansprüche 1 bis 6, wobei das Steuersystem ferner ein Datenverbindungsmodul für eine Fernkommunikation und -steuerung der Vorrichtung (1) umfasst.

8. Vernebelungsvorrichtung (2) nach Anspruch 7, wobei die Vorrichtung (1) ferner eine ferngesteuerte Prüf- und Steuervorrichtung zur Verarbeitung des mindestens einen Signals umfasst, wobei die ferngesteuerte Vorrichtung über das Datenverbindungsmodul mit der Vorrichtung (1) verbunden ist.

9. Vernebelungsvorrichtung (2) nach einem der Ansprüche 1 bis 8, wobei das Verbindungselement (6) der Vorrichtung (1) durch einen Verbindungsschlauch (14) verbunden ist.1

10. Vernebelungsvorrichtung (2) nach Anspruch 2 und 9, wobei der Verbindungsschlauch (14) der Vorrichtung (1) einen Membranfilter umfasst, um den Drucksensor vor möglichen vernebelten Rückständen wie Medikamenten oder Hilfsstoffen zu schützen.

11. Vernebelungsvorrichtung (2) nach einem der Ansprüche 1 bis 10, wobei die Vorrichtung (1) ferner einen Netzwerkanschluss (15) zum Verbinden der Vorrichtung (1) mit einem externen System umfasst.

12. Vernebelungsvorrichtung (2) nach einem der Ansprüche 1 bis 11, wobei die Vorrichtung (1) ferner mindestens eine Konfigurationstaste (5) umfasst.

13. Vernebelungsvorrichtung (2) nach einem der Ansprüche 1 bis 12, wobei die Vorrichtung (1) ferner ein Batteriesystem umfasst.

## Revendications

1. Dispositif de nébulisation (2) comprenant un appareil (1) pour vérifier et contrôler la respiration d'un patient, ledit appareil (1) comprenant:
- un élément de connexion (6) pour connecter ledit appareil (1) avec les voies respiratoires d'un patient;
- un corps principal (3) pourvu d'un dispositif d'affichage intégré (4);
- un ou plusieurs capteurs adaptés pour détecter en continu pendant la thérapie par inhalation une variation d'au moins une grandeur physique associée à un flux d'air inspiré et/ou expiré par le patient, passant par ledit élément de connexion (6) et susceptible d'émettre au moins un signal lié à ladite variation;
- un système de contrôle intégré dans l'appareil et configuré pour recevoir ledit signal pour effectuer une vérification de correspondance dudit au moins un signal avec au moins un paramètre prédéfini stocké dans ledit système de contrôle, et émettre un signal d'erreur en cas de non-correspondance entre ledit au moins un signal et ledit au moins un paramètre prédéfini, ledit paramètre prédéfini étant obtenu par ledit système de contrôle par un examen initial de la respiration du patient par une analyse d'une pluralité de respirations, ledit paramètre prédéfini étant comparé au cours de chaque administration unique dudit dispositif de nébulisation (2) au signal détecté par lesdits un ou plusieurs capteurs.

2. Dispositif de nébulisation (2) selon la revendication 1, dans lequel lesdits un ou plusieurs capteurs dudit appareil (1) comprennent au moins un capteur de pression adapté pour détecter une pression du flux d'air inspiré et/ou expiré par un patient, passant par ledit élément de connexion (6) et susceptible d'émettre au moins un signal lié à ladite pression.

3. Dispositif de nébulisation (2) selon la revendication 2, dans lequel ledit au moins un capteur de pression est du type pressostat différentiel.

4. Dispositif de nébulisation (2) selon l'une quelconque des revendications 1 à 3, dans lequel ledit appareil (1) comprenant en outre un système de fixation rapide par clip (7), adapté pour permettre une liaison par encliquetage (7) avec ledit dispositif de nébulisation (2).

5. Dispositif de nébulisation (2) selon la revendication 4, dans lequel ledit système de fixation rapide (7) assure une liaison par encliquetage par une partie en forme de « C » de fixation élastique.

6. Dispositif de nébulisation (2) selon la revendication 4, dans lequel ledit système de fixation rapide (7) fournit une sangle de fixation en matériau élastique ou Velcro.

7. Dispositif de nébulisation (2) selon l'une quelconque des revendications 1 à 6, dans lequel ledit système de contrôle comprend en outre un module de connexion de données pour une communication et un contrôle à distance dudit appareil (1).

8. Dispositif de nébulisation (2) selon la revendication 7, dans lequel ledit appareil (1) comprenant en outre un dispositif distant de vérification et de contrôle pour le traitement dudit au moins un signal, ledit dispositif distant étant connecté audit appareil (1) par ledit module de connexion de données.

9. Dispositif de nébulisation (2) selon l'une quelconque des revendications 1 à 8, dans lequel ledit élément de connexion (6) dudit appareil (1) est connecté par un tube de connexion (14).

10. Dispositif de nébulisation (2) selon les revendications 2 et 9, dans lequel ledit tube de connexion (14) dudit appareil (1) comprend un filtre à membrane pour protéger ledit capteur de pression d'éventuels résidus nébulisés tels que des médicaments ou des excipients.

11. Dispositif de nébulisation (2) selon l'une quelconque des revendications 1 à 10, dans lequel ledit appareil (1) comprenant en outre un port de connexion réseau (15) pour connecter ledit appareil (1) à un système externe.

12. Dispositif de nébulisation (2) selon l'une quelconque des revendications 1 à 11, dans lequel ledit appareil (1) comprenant en outre au moins un bouton de configuration (5).

13. Dispositif de nébulisation (2) selon l'une quelconque des revendications 1 à 12, dans lequel ledit appareil (1) comprenant en outre un système d'alimentation par batterie.
